Europäisches Patentamt

(19) European Patent Office

Office européen des brevets

(11) Numéro de publication: **0 188 963**

**B1**

(12) **FASCICULE DE BREVET EUROPÉEN**

(45) Date de publication du fascicule du brevet:
14.12.88

(21) Numéro de dépôt: **85402610.1**

(22) Date de dépôt: **24.12.85**

(51) Int. Cl.⁴: **C 08 F 220/06,** C 08 F 220/34 //
(C08F220/06, 220:34),
(C08F220/34, 220:06)

(54) **Résines hydro-absorbantes, leur procédé de fabrication et leur application à l'obtention d'articles capables d'absorber des fluides aqueux.**

(30) Priorité: **27.12.84 FR 8419878**

(43) Date de publication de la demande:
**30.07.86 Bulletin 86/31**

(45) Mention de la délivrance du brevet:
**14.12.88 Bulletin 88/50**

(84) Etats contractants désignés:
**AT BE CH DE FR GB IT LI LU NL SE**

(56) Documents cité:
**US-A-3 634 366**
**US-A-4 212 782**

(73) Titulaire: **NORSOLOR S.A., Tour Aurore Place des Reflets, F-92080 Paris la Defense Cédex 5 (FR)**

(72) Inventeur: **Cretenot, Claude- Lise, 1 rue de la Pinède, F-60550 Verneuil en Halatte (FR)**
Inventeur: **Wiegert, Bernard, 2 rue Frédéric Mistral Brenouille, F-60870 Rieux (FR)**

(74) Mandataire: **Dubost, Thierry, c/o NORSOLOR Service Propriété Industrielle B.P. 57, F-62670 Mazingarbe (FR)**

EP 0 188 963 B1

## Description

La présente invention concerne des résines hydroabsorbantes, leur procédé de fabrication et leur application à l'obtention d'articles capables d'absorber des fluides aqueux.

On connaît par le brevet européen n° 055 728 des copolymères ayant une capacité élevée d'absorption et de rétention d'eau et comprenant:

a) de 65 à 95 % en poids d'acide acrylique, et

b) de 5 à 35 % en poids d'un monomère choisi parmi les méthacrylates d'alkyle dont le groupe alkyle a de 1 à 30 atomes de carbone, le méthacrylate de cyclohexyle, l'acrylate de phényle, les acrylates de phénoxyalkyle dont le groupe alkyle a de 2 à 6 atomes de carbone, et les (méth)acrylates de dialkylaminoalkyle dont le groupe alkyle a de 2 à 6 atomes de carbone et dont le groupe dialkyle a de 2 à 8 atomes de carbone.

Ces copolymères, pour lesquels le monomère préféré est le méthacrylate de lauryle, le méthacrylate de 2-hydroxyéthyle ou l'acrylate de phénoxyéthyle, peuvent comprendre un agent réticulant.

On connaît par le brevet américain n° 3 634 366 un procédé de polymérisation, en l'absence de tout initiateur et sous atmosphère inerte:

- d'un monomère choisi parmi les acides aliphatiques monobasiques et dibasiques $\alpha, \beta$ insaturés, ce monomère pouvant être l'acide acrylique, et

- d'un monomère insaturé possédant un groupement ami ne tertiaire et susceptible de quaternisation, ce monomère pouvant être choisi dans un groupe comprenant le diméthylaminométhyl méthacrylate ou le diméthylaminoéthyl méthacrylate.

Selon ce document le rapport molaire du monomere acide au monomère insaturé aminé est compris entre 0,8 et 1,2; la polymérisation est effectuée pendant une durée de 1 à 10 heures et à une température de 0° à 150°C. Les produits obtenus sont utiles comme conditionneurs de soi et agents de floculation.

On connaît également par le brevet américain n° 4 212 782 un polymère hydrosoluble, amphotère en milieu aqueux, d'un sel hydrosuble de l'acide méthacrylique et d'un 2-monoalkylaminoéthyl méthacrylate dont le groupe alkyle possède au plus 6 atomes de carbone, le rapport molaire des deux monomères étant compris entre 2 : 3 et 3 : 2.

Le but de la présente invention réside dans la mise au point de produits ayant une capacité très élevée d'absorption et de rétention d' eau, pure ou saline, c'est-à-dire contenant une quantité notable d'ions minéraux. La présente invention est basée sur la découverte surprenante que:

- l'enseignement du brevet européen n° 055 728, à savoir la nature du monmère préféré et la proportion d'acide acrylique comprise entre 65 et 95 % en poids, ne permet pas de résoudre le problème d'une forte capacité d'absorption et de rétention d'eau pure ou saline,

- un copolymère d'acide acrylique et de diméthylaminoéthyl méthacrylate tel que décrit dans le brevet américain n° 3 634 366 ne possède aucune capacité d'absorption et de rétention d'eau pure ou saline,

- un copolymère d'acide méthacrylate et de méthylaminoéthyl acrylate tel que décrit dans le brevet américain n° 4 212 782 ne possède qu'une faible capacité d'absorption et de rétention d'eau pure ou saline.

En conséquence un premier objet de la présente invention est un résine hydroabsorbante caractérisée en ce qu'elle comprend de 40 à 60 % en moles d'acide acrylique, le cas échéant au moins partiellement salifié, et de 60 à 40 % en moles d'au moins un dialkylaminoalkyl acrylate dans lequel chaque groupe alkyle a de 1 à 4 atomes de carbone, le cas échéant au moins partiellement salifié ou quaternisé.

Par acide acrylique le cas échéant au moins partiellement salifié, on entend que l'acide acrylique peut être en partie remplacé par au moins l'un de ses sels d'ammonium ou alcalin ou encore alcalino-terreux. Par dialkylaminoalkyl acrylate le cas échéant au moins partiellement salifié ou quaternisé, on entend que ledit dialkylaminoalkyl acrylate peut être en partie remplacé par au moins un de ses sels, tel que son sel d'ammonium quaternaire.

Les résines hydroabsorbantes selon l'invention sont remarquables en ce qu'elles possèdent la capacité d'absorber et de retenir non seulement de 100 à 500 fois leur poids d'eau pure mais également de 20 à 60 fois leur poids d'eau saline, en particulier d'eau contenant des sels monovalents ou divalents d'un métal alcalin ou alcalino-terreux tel que par exemple chlorure de sodium, dichlorure de calcium, phosphate de potassium ou de sodium, sulfate de potassium ou de magnésium, nitrate de potassium ou d'ammonium.

En outre la demanderesse a trouvé de manière surprenante que les propriétés mécaniques, en particulier la résistance à la compression, des résines hydroabsorbantes selon l'invention peuvent être améliorées de façon très importante lorsque lesdites résines comprennent en outre au moins un agent réticulant en quantité suffisante, plus particulièrement en quantité au moins égale à 1 % en poids et de préférence au plus égale à 5 % en poids par rapport à la somme des monomères constitutifs desdites résines.

Comme agent réticulant on peut utiliser par exemple:

1) des composés ayant au moins deux doubles liaisons polymérisables et,

2) des composés ayant au moins une double liaison liaison polymérisable et au moins un groupe fonctionnel réactif avec au moins l'un des monomères.

Des exemples des composés répertoriés en premièrement ci-dessus, ayant au moins deux doubles liaisons polymérisables, sont:

a) les composés di- ou polyvinyliques, tels que notamment le divinylbenzène, le divinyltoluène, le divinylxylène, l'éther divinylique, la divinylcétone et le trivinylbenzène,

b) les di- ou polyesters d'acides mono- ou polycarboxyliques non saturés avec des polyols, tels que les esters des acides di- ou tri(méth)acryliques avec des polyols (tels que l'éthylène glycol, le triméthylolpropane, le glycérol, les polyoxyéthylèneglycols, les polyoxypropylèneglycols), les polyesters non saturés (que l'on peut obtenir par réaction de l'un quelconque des polyols précités avec un acide non saturé tel que l'acide maléique), les esters d'acide di- ou tri-(meth)acrylique que l'on peut obtenir par réaction d'un polyépoxide avec l'acide (méth)acrylique),

c) les bis (méth)acrylamides tels que la N,N-méthylène-bis-acrylamide,

d) les esters carbamyliques que l'on peut obtenir en faisant réagir des polyisocyanates (tels que le toluène diisocyanate, l'hexaméthylène diisocyanate, le 4,4'-diphénylméthanediisocyanate, les prépolymères contenant un groupe NCO obtenus en faisant réagir un tel diisocyanate avec des composés contenant des atomes d'hydrogène actifs) avec des monomères contenant des groupes hydroxyles. De tels esters sont notamment ceux des acides di(méth)acryliques que l'on peut obtenir en faisant réagir les diisocyanates précités avec le (méth)acrylate d'hydroxyéthyle,

e) les éthers di- ou poly(méth)allyliques de polyols tels que les alkylènes glycols, le glycérol, les polyalkylèneglycols, les polyoxyalkylènepolyols, les hydrates de carbones, tels que l'éther diallylique du polyéthylène glycol, l'amidon allylé et la cellulose allylée,

f) les esters di- ou polyallyliques d'acides polycarboxyliques tels que le phthalate de diallyle, l'adipate de diallyle, et

g) les esters d'acides mono- ou polycarboxyliques non saturés avec des éthers mono(méth)allyliques de polyols, tels que l'ester de l'acide (méth)acrylique avec l'éther monoallylique du polyéthylène glycol.

Les composés du type deuxièmement précités ayant au moins une double liaison polymérisable et au moins un groupe fonctionnel réactif avec au moins l'un des monomères sont les composés éthyléniquement insaturés contenant au moins un groupe réactif avec les groupes carboxyle, anhydride carboxylique, hydroxyle, amine ou amide. Des exemples de ces composes sont la N-méthylol(méth)acrylamide, le (méth)acrylate de glycidyle.

Les résines hydroabsorbantes ainsi obtenues possèdent en outre l'avantage considérable, notamment pour des applications dans le domaine agricole où elles sont susceptibles d'être soumises au rayonnement solaire, de conserver leurs propriétés d'absorption et de rétention d'eau après une exposition prolongée au rayonnement ultraviolet.

Un second objet de la présente invention consiste en un procédé de fabrication des résines hydro-absorbantes décrites précédemment, par copolymérisation d'un mélange constitué de 40 à 60 % en moles d'acide acrylique, le cas échéant au moins partiellement salifié, et de 60 à 40 % en moles d'au moins un dialkylaminoalkyl acrylate, le cas échéant au moins partiellement salifié ou quaternisé, en présence d'au moins un générateur de radicaux libres,

- soit en solution aqueuse, pendant une durée comprise entre 1 et 240 minutes, à une température comprise entre 2° et 98°C,

- soit en émulsion inverse, c'est-à-dire en dispersant les comonomères, le cas échéant en mélange avec de l'eau, dans une phase organique non miscible à l'eau et aux monomères, en présence d'au moins un agent émulsifiant. Cette technique est couramment désignée sous le terme polymérisation dans une émulsion "eau dans l'huile". Des exemples de phase organique sont des hydrocarbures aliphatiques (pentane, hexane, heptane, décane, etc. ou leurs mélanges) ou aromatiques (benzène, toluène, xylènes ou leurs mélanges). Le rapport pondéral eau/monomères est généralement inférieur ou égal à 3. Le rapport pondéral de la phase organique à la phase contenant les monomères est de préférence compris entre 2 et 5. Des agents émulsifiants utilisables sont des agents tensio-actifs non ioniques lipophiles (le cas échéant en mélange avec des agents tensio-actifs non ioniques hydrophiles), tels que par exemple le monooléate de sorbitan (seul ou en mélange avec le monoléate de sorbitan éthoxylé), le monostéarate de sorbitan, les alkylphénols éthoxylés. Le rapport pondéral de l'agent émulsifiant à la phase organique est de préférence compris entre 0,03 et 0,20. La durée de la réaction est comprise entre 30 et 360 minutes, la température de réaction est comprise entre 20° et 80°C.

Le plus souvent la copolymérisation sera effectuée sous pression atmosphérique. La température et la durée de polymérisation seront choisies l'une en fonction de l'autre, la durée étant d'autant plus longue que la température est plus basse et vice-versa. A l'issue de la durée de réaction préconisée, la conversion du mélange est généralement très proche de 100 %. Tout moyen capable de générer des radicaux libres pourra être utilisé dans le cadre du procédé selon l'invention. Il pourra s'agir notamment de microondes, de rayonnements béta, gamma ou ultraviolet, ou encore d'initiateurs chimiques. Dans ce dernier cas l'initiateur de polymérisation peut être notamment choisi parmi les persulfates, les peroxydes, les hydroperoxydes et les composés diazoïques; lorsqu'un persulfate de métal alcalin est choisi, il peut être utilisé en combinaison avec un réducteur choisi parmi les polyhydrophénols, le sulfite et le bisulfite de sodium, le diméthylaminopropio nitrile, les diazomercaptans et les ferricyanures. L'initiateur et le cas échéant, le réducteur, sont utilisables à raison de 0,1 a 2 % chacun en poids par rapport à l'ensemble des monomères présents. Selon un mode particulier de réalisation de l'invention, la polymérisation peut être effectuée selon un procédé discontinu jusqu'à ce que la teneur en extrait sec dans le milieu atteigne environ 20 % en poids.

Enfin un troisième objet de la présente invention consiste en des articles capables d'absorber les fluides aqueux, comprenant au moins une résine hydroabsorbante telle que décrite précédemment. De tels articles peuvent trouver des usages variés dans le domaine de l'hygiène (couches pour bébés et incontinents) en raison de leur capacité à absorber le sang et/ou l'urine, ainsi que dans le domaine de l'agriculture (rétention de l'eau dans les zones géographiques faiblement arrosées) et de l'industrie (agents déshydratants). Ils peuvent se présenter notamment sous forme de poudre ou de granulés.

Les exemples ci-après sont donnés à titre illustratif de la présente invention.

### Exemple 1

33,5 g d'acide acrylique et 66,5 g d'acrylate de diméthylaminoéthyle sont mis en solution dans 400 g d'eau en présence d'un système catalytique comprenant 1 g de persulfate d'ammonium et 1 g de métabisulfite de sodium. La polymérisation est alors effectuée à la température de 80°C pendant 120 minutes. Le copolymère résultant est alors refroidi puis séché dans une étuve ventilée à 60°C pendant 24 heures et enfin séché sous vide partiel (100 mm Hg) à 30°C. Après broyage sous forme de poudre, le produit obtenu est soumis aux deux tests suivants:

Capacité d'absorption et de rétention d'eau:
Le produit est additionné progressivement d'eau pure ou d'eau saline comprenant 1 % de chlorure de sodium jusqu'à saturation. Les poids d'eau absorbés pour parvenir à saturation sont les suivants:
- eau pure: 300 fois le poids de copolymère.
- eau saline: 55 fois le poids de copolymère.
Résistance à la compression:
On fait gonfler 1 g du copolymère en poudre dans 100 g d'eau déminéralisée. Après essorage pour enlever toute trace d'eau non absorbée, on place le gel obtenu dans un bécher de diamètre intérieur 140 mm, où il occupe une hauteur de 50 mm. On pose alors sur le gel un poids de 100 g puis on mesure la profondeur de pénétration de ce poids dans le gel lorsqu'elle est stabilisée. On note une profondeur de 40 mm.

### Exemple 2

On répète les opérations de l'exemple 1 en polymérisant un mélange de 33 g d'acide acrylique, 65,5 g d'acrylate de diméthylaminoéthyle et 1,5 g de diméthacrylate d'éthylène glycol. Dans ce cas les propriétés mesurées en effectuant les deux tests décrits à l'exemple 1 sont les suivantes:
Capacité d'absorption et de rétention d'eau:
- eau pure: 130 fois le poids de copolymère.
- eau saline: 40 fois le poids de copolymère.
Résistance à la compression:
Profondeur de pénétration : 10 mm

### Exemple 3

32,8 g d'acide acrylique, 65,2 g d'acrylate diméthylaminoéthyle et 2 g de diméthacrylate d'éthylène glycol sont mis en solution dans 400 g d'eau en présence de 1 g de persulfate d'ammonium et de 1 g de métabisulfite de sodium. La polymérisation est alors effectuée à la température de 85°C pendant 180 minutes. Le copolymère résultant, traité et broyé comme à l'exemple 1 est soumis au test d'absorption et de rétention d'eau. Les résultats sont les suivants:
- eau pure: 460 fois le poids de copolymère
- eau saline: 60 fois le poids de copolymère
D'autre part le gel obtenu après absorption d'eau pure jusqu'à saturation est soumis pendant 1 heure au rayonnement ultraviolet d'une lampe de puissance 4 watts. A l'issue de ce traitement, le gel est essoré pour enlever toute trace d'eau non absorbée puis pesé de manière à mesuré sa capacité de rétention d'eau après vieillissement. Celle-ci égale à 460 fois.

### Exemple 4 (Comparatif)

On répète les opérations de l'exemple 1, à la seule exception suivante: l'acrylate de diméthylaminoéthyle est remplacé par le méthacrylate de diméthylaminoéthyle. Le polymère obtenu dans ces conditions se révèle incapable d'absorber l'eau pure ou l'eau saline.

### Exemple 5 (Comparatif)

On répète les opérations de l'exemple 1, à la seule exception suivante l'acide acrylique est remplacé par l'acide méthacrylique. Le polymère obtenu dans ces conditions présente les capacités d'absorption et de rétention suivantes, mesurées selon le test décrit à l'exemple 1:
- eau pure: 60 fois
- eau saline: 15 fois.

### Revendications

1. Résine hydroabsorbante, caractérisée en ce qu'elle comprend de 40 à 60 % en moles d'acide acrylique, le cas échéant au moins partiellement salifié, et de 60 à 40 % en moles d'au moins un dialkylaminoalkyl acrylate dans lequel chaque groupe alkyle a de 1 à 4 atomes de carbone, le cas échéant au moins partiellement salifié ou quaternisé.

2. Résine hydroabsorbante selon la revendication 1, caractérisée en ce qu'elle comprend en outre au moins un agent réticulant en quantité comprise entre 1 et 5 % en poids par rapport à la somme des monomères.

3. Procédé de fabrication d'une résine hydroabsorbante selon ta revendication 1, par copolymérisation d'un mélange constitué de 40 à 60 % en moles d'acide acrylique, le cas échéant au moins partiellement salifié, et de 60 à 40 % en moles d'au moins un dialkylaminoalkyl acrylate, le cas échéant au moins partiellement salifié ou quaternisé, en présence d'au moins un générateur de radicaux libres, la copolymérisation étant effectuée soit en solution aqueuse, soit en émulsion inverse.

4. Procédé selon la revendication 3, la copolymérisation étant effectuée en solution aqueuse, caractérisé en ce que la température est comprise entre 2° et 98°C et en ce que la durée est comprise entre 1 et 240 minutes.

5. Procédé selon la revendication 3, la copolymérisation étant effectuée en émulsion inverse, en dispersant le mélange des comonomères dans une phase organique non miscible auxdits comonomères en présence d'au moins un agent émulsifiant, caractérisé en ce que la température est comprise entre 20° et 80°C et en ce que la durée est comprise entre 30 et 360 minutes.

6. Procédé selon la revendication 5, caractérisé en ce que l'agent émulsifiant est un agent tensioactif non ionique lipophile.

7. Procédé selon l'une des revendications 5 et 6, caractérisé en ce que l'agent émulsifiant est utilisé dans un rapport pondéral à la phase organique compris entre 0,03 et 0,20.

8. Procédé selon l'une des revendications 3 à 7, caractérisé en ce que le générateur de radicaux libres est choisi parmi les microondes, les rayonnements béta, gamma et ultraviolet, les peroxydes, les hydroperoxydes, les persulfates et les composés diazoïques.

9. Articles capables d'absorber des fluides aqueux, caractérisés en ce qu'ils comprennent au moins une résine hydroabsorbante selon l'une des revendications 1 et 2.

## Patentansprüche

1. Wasserabsorbierendes Harz, dadurch gekennzeichnet, daß es 40 bis 60 Mol-% Acrylsäure, gegebenen falls zumindest teilweise in Form ihrer Salze, und 60 bis 40 Mol-% mindestens eines Dialkylaminoalkylacrylats, bei dem jede Alkylgruppe 1 bis 4 Kohlenstoffatome besitzt und das gegebenenfalls zumindest teilweise in Form seiner Salze oder als quaternäre Verbindung vorliegt, enthält.

2. Wasserabsorbierendes Harz nach Anspruch 1, dadurch gekennzeichnet, daß es weiters mindestens ein Vernetzungsmittel in einer Menge zwischen 1 und 5 Gew.-% bezogen auf die Summe der Monomere enthält.

3. Verfahren zur Herstellung eines wasserabsorbierenden Harzes nach Anspruch 1 durch Copolymerisation einer Mischung bestehend aus 40 bis 60 Mol-% Acrylsäure, gegebenenfalls zumindest teilweise in Form ihrer Salze, und 60 bis 40 Mol-% mindestens eines Dialkylaminoalkylacrylats, gegebenenfalls zummindest teilweise in Form seiner Salze oder als quaternäre Verbindung, in Gegenwart eines Mittels zur Erzeugung freier Radikale, wobei die Copolymerisation entweder in wässriger Lösung oder in Invertemulsion erfolgt.

4. Verfahren nach Anspruch 3, bei welchem die Copolymerisation in wässriger Lösung erfolgt, dadurch gekennzeichnet, daß die Temperatur zwischen 2° und 98°C liegt und die Dauer zwischen 1 und 240 Minuten beträgt.

5. Verfahren nach Anspruch 3, bei welchem die Copolymerisation in Invertemulsion erfolgt, indem die Mischung der Comonomere in Gegenwart mindestens eines Emulgators in eine organische, mit den genannten Comonomeren nicht mischbare Phase dispergiert wird, dadurch gekennzeichnet, daß die Temperatur zwischen 20° und 80°C liegt und die Dauer zwischen 30 und 360 Minuten beträgt.

6. Verfahren nach Anspruch 5, dadurch gekennzeichnet, daß der Emulgator ein nicht ionischer, lipophiler oberflächenaktiver Stoff ist.

7. Verfahren nach Anspruch 5 oder 6, dadurch gekennzeichnet, daß der Emulgator in einem Gewichtsverhältnis zwischen 0,03 und 0,20 zur organischen Phase eingesetzt wird.

8. Verfahren nach einem der Ansprüche 3 bis 7, dadurch gekennzeichnet, daß das Mittel zur Erzeugung freier Radikale ausgewählt ist aus der Gruppe bestehend aus den Mikrowellen, den Beta-, Gamma- und Ultraviolettstrahlungen, den Peroxiden, den Hydroperoxiden, den Persulfaten und den Diazoverbindungen.

9. Gegenstände, die fähig sind, wässrige Fluide zu absorbieren, dadurch gekennzeichnet, daß sie mindestens ein wasserabsorbierendes Harz nach Anspruch 1 oder 2 enthalten.

## Claims

1. Water-absorbing resin, characterized in that it comprises from 40 to 60 % on a molar basis of acrylic acid, at least partially in salt form if appropriate, and from 60 to 40 % on a molar basis of at least one dialkylamino alkyl acrylate in which each alkyl group contains from 1 to 4 carbon atoms, at least partially in salt form or quaternized if appropriate.

2. Water-absorbing resin according to Claim 1, characterized in that it additionally comprises at least one cross-linking agent in a quantity of between 1 and 5 % by weight based on the total of the monomers.

3. Process for the manufacture of a water-absorbing resin according to claim 1, by copolymerization of a mixture consisting of 40 to 60 % on a molar basis of acrylic acid, at least partially in salt form if appropriate, and from 60 to 40 % on a molar basis of at least one dialkylaminoalkyl acrylate, at least partially in salt form or quaternized if appropriate, in the presence of at least one freeradical generator, the copolymerization being performed either in aqueous solution or in an inverse emulsion.

4. Process according to claim 3, the copolymerization being performed in aqueous solution, characterized in that the temperature is between 20 and 98°C and that the time is between 1 and 240 minutes.

5. Process according to claim 3, the copolymerization being performed in an inverse emulsion, by dispersing the mixture of the comonomers in an organic phase which is immiscible with the said comonomers in the presence of at least one emulsifying agent, characterized in that the temperature is between 20° and 80°C and that the time is between 30 and 360 minutes.

6. Process according to claim 5, characterized in that the emulsifying agent is a lipophilic nonionic surface-active agent.

7. Process according to either of claims 5 and 6, characterized in that the emulsifying agent is used in a weight ratio of between 0.03 and 0.20 to the organic phase.

8. Process according to one of claims 3 to 7, characterized in that the free-radical generator is chosen from microwaves, beta, gamma and ultraviolet radiations, peroxides, hydroperoxides, persulphates and diazo compounds.

9. Articles capable of absorbing aqueous liquids, characterized in that they comprise at least one water-absorbing resin according to either of claims 1 and 2.